# EUROPEAN PATENT APPLICATION

(11) **EP 4 417 303 A1**
(43) Date of publication of application: **21.08.2024**
(21) Application number: 22880288.0
(22) Date of filing: 11.10.2022
(51) Int. Cl.: B01J 23/887, B01J 23/881, B01J 23/78, B01J 23/745, B01J 23/83, B01J 37/00, B01J 37/08, C07C 5/32, C07C 5/333, C07C 15/46

(54) **ETHYLBENZENE DEHYDROGENATION CATALYST, PREPARATION METHOD THEREFOR, AND USE THEREOF**

(30) Priority: 11.10.2021 CN 202111183786
(71) Applicant: China Petroleum & Chemical Corporation, Beijing 100728 (CN); Shanghai Research Institute of Petrochemical Technology, SINOPEC, Shanghai 201208 (CN)
(72) Inventor: ZENG, Tieqiang, Shanghai 201208 (CN); MIAO, Changxi, Shanghai 201208 (CN); SONG, Lei, Shanghai 201208 (CN); WEI, Chunling, Shanghai 201208 (CN); ZHU, Min, Shanghai 201208 (CN); ZHANG, Zhengpai, Shanghai 201208 (CN); XU, Yongfan, Shanghai 201208 (CN); ZHA, Kaiwen, Shanghai 201208 (CN)
(74) Representative: karo IP
(86) International application number: PCT/CN2022/124556
(87) International publication number: WO 2023/061351

(57) **Abstract**

The present invention relates to an ethylbenzene dehydrogenation catalyst, a preparation method therefor, and the use thereof. The catalyst comprises Fe₂O₃, K₂O, CeO₂, MoO₃ and CaO, wherein the exposed crystal face area of CeO₂(100) accounts for 60% or more of the total exposed crystal face area of CeO₂. The catalyst of the present invention is used in a reaction for preparing styrene by means of dehydrogenating ethylbenzene at a low water ratio, and has high activity and stability.

## Description

### Technical Field

The present invention relates to an ethylbenzene dehydrogenation catalyst, in particular to a catalyst for preparing styrene by dehydrogenating ethylbenzene with a low steam/ethylbenzene ratio and preparation method therefor, and use thereof.

### Background of Art

Styrene is an important bulk chemical raw material, mainly used as an important raw material in the synthetic rubber and plastics industries. It has a wide range of uses and possesses an important position in the national economy.

There are currently two main routes for producing styrene from ethylbenzene in the world: one is the catalytic dehydrogenation of ethylbenzene to produce hydrogen gas and styrene, and the other is the co-production of PO/SM by co-oxidation. The catalytic dehydrogenation of ethylbenzene has always been the leading technical route for the production of styrene, and its production capacity accounts for more than 85% of the total styrene production capacity.

Ethylbenzene dehydrogenation is a strong endothermic reversible reaction in which the number of molecules increases. High temperature and low pressure are conducive to the reaction. In industry, a large amount of high-temperature water vapor is usually introduced to facilitate the production of the target product styrene. Water vapor plays many important roles in the reaction: providing the heat required for the reaction; reducing the partial pressure of ethylbenzene and promoting the chemical balance to move toward styrene; eliminating carbon deposited on the catalyst surface through the water-vapor shift reaction; and oxidizing the catalyst surface to maintain catalyst activity. However, the use of a large amount of superheated steam as a dehydrogenation medium makes the process consume a lot of energy, produces a large volume of condensate, costs a lot in process equipments and has a high production cost. Therefore, the ethylbenzene dehydrogenation process aims to achieve a higher styrene yield with a lower steam/ethylbenzene ratio (also called "water ratio", i.e., the mass ratio of water vapor to ethylbenzene in the feed). Operating with a low steam/ethylbenzene ratio is one of the important measures to save energy and reduce consumption. It has become an urgent need for styrene enterprises to develop styrene catalysts suitable for low steam/ethylbenzene ratios and improve catalyst activity, selectivity and stability, thereby achieving energy conservation and consumption reduction, as well as lower production costs in styrene production facilities.

Most of the existing styrene catalysts are mainly composed of Fe-K-Ce, with Fe-K oxides as the main catalyst and Ce as the main promoter, and also contain oxides of Mg, Mo, W, and Ca as structural stabilizers and electronic auxiliaries. The ethylbenzene dehydrogenation catalysts for low steam/ethylbenzene ratios that have been disclosed so far mainly use methods such as improving the structural stability of catalysts and modifying the surface properties of catalysts to improve the stability, activity and selectivity of catalysts under low steam/ethylbenzene ratio conditions.

CN106582678A discloses that on the basis of a Fe-K-Ce-W catalyst, oxides of Ba, Sn and rare earth (Sm, Eu, Gd) are introduced to stabilize the active phase of the catalyst and realize the high activity and the high stability of the catalyst under low steam/ethylbenzene ratio conditions.

CN1981929A discloses, on the basis of a Fe-K-Ce-W catalyst system, adding at least two light rare earth oxide additives (at least two of La, Pr, Nd, and Sm) other than cerium, and at the same time adding at least one metal oxide of Ca, Mg, Ba, B, Sn, Pb, Cu, Zn, Ti, Zr and Mo oxides, resulting in improving the stability and activity of the low-K catalyst under low steam/ethylbenzene ratio conditions.

Today's styrene production technology is developing in the direction of large-scale equipment and comprehensive energy utilization. Developing an ethylbenzene dehydrogenation catalyst suitable for operation under low steam/ethylbenzene ratio conditions, with higher activity, selectivity, and better stability has always been the direction of researchers' efforts.

### Summary of the Invention

One of the technical problems to be solved by the present invention is a problem present in the existing ethylbenzene dehydrogenation technology that the catalyst has lower activity and worse stability in the condition of low steam/ethylbenzene ratio, and therefore there is provided a new ethylbenzene dehydrogenation catalyst for preparing styrene. The catalyst has high activity and stability for the ethylbenzene dehydrogenation reaction under a low steam/ethylbenzene ratio.

The second technical problem to be solved by the present invention is to provide a method of preparing an ethylbenzene dehydrogenation catalyst for preparing styrene, corresponding to solving the first technical problem.

The third technical problem to be solved by the present invention is to provide use of an ethylbenzene dehydrogenation catalyst for preparing styrene, corresponding to solving the first technical problem, in the production of styrene by ethylbenzene dehydrogenation at a low steam/ethylbenzene ratio.

In order to solve the above first technical problem, the technical solutions adopted by the present invention are as follows:
The first aspect of the present invention provides a catalyst, wherein the catalyst can contain Fe₂O₃, K₂O, CeO₂, MoO₃, and CaO; wherein the area of CeO₂ (100) exposed crystal face may comprise 60% or higher of the area of CeO₂ total exposed crystal face.

In the above-mentioned technical solution, the H₂-TPR main reduction peak position of the catalyst can be 570-620°C.

In the above-mentioned technical solution, the catalyst may include the following components in mass fraction based on the total mass of the catalyst:
(a) 60%-85% of Fe₂O₃;
(b) 6%-14% of K₂O;
(c) 6%-14% of CeO₂;
(d) 0.5%-5% of MoO₃;
(e) 0.3%-7% of CaO.

In the above-mentioned technical solution, preferably the catalyst may contain 0.01%-2.0% Na₂O in mass fraction.

In the above-mentioned technical solution, the catalyst may also contain 0.01%-2.0% other metal oxide(s), such as TiO₂ in mass fraction.

In the above-mentioned technical solution, preferably, the area of CeO₂ (100) exposed crystal face may comprise 70% or higher; for example but not limited to 70%, 75%, 80%, 85%, 87%, 90%, 92%, 95%, 97%, 99% and the like of the area of CeO₂ total exposed crystal face.

In the above-mentioned technical solution, the H₂-TPR main reduction peak position of the catalyst can be 570-620°C; the H₂-TPR main reduction peak position of the catalyst is for example but not limited to 570°C, 580°C, 585°C, 590°C, 595°C, 600°C, 605°C, 610°C, 615°C, 620°C and the like.

In order to solve the above second technical problem, the technical solutions adopted by the present invention are as follows:
The second aspect of the present invention provides the above-mentioned method of preparing an ethylbenzene dehydrogenation catalyst, comprising the following steps:
mixing a Fe source, a Ce source, a Mo source, a Ca source, optionally a first K source, and optionally a pore-forming agent evenly, then adding an alkaline solution, letting stand for reaction, shaping, and calcining to produce the catalyst;
wherein the K₂O in the catalyst can derive from the first K source and/or the alkaline solution.

In the above-mentioned technical solution, the alkaline solution can be an aqueous solution of potassium hydroxide and/or sodium hydroxide, preferably an aqueous solution of sodium hydroxide and potassium hydroxide. In case that the alkaline solution is an aqueous solution of sodium hydroxide and potassium hydroxide, the mass ratio of sodium hydroxide as Na₂O to potassium hydroxide as K₂O is preferably 1 :2-23. The OH group concentration of the alkaline solution can be 1 mol/L - 8 mol/L. In the method of preparing the catalyst, the alkaline solution is used in such an amount that is sufficient to ensure that the cerium source is dissolved in the liquid phase for the reaction.

In the above-mentioned technical solution, the K₂O in the catalyst can derive from the first K source and/or the alkaline solution, preferably, the K₂O in the catalyst can at leaat partially derive from the alkaline solution. Preferably, at least 50% of the K₂O in the catalyst can derive from an alkaline solution containing K, and the remaining part derives from the first K source. The K₂O in the catalyst can also all derive from the alkaline solution containing K, instead of using the first K source.

In the above-mentioned technical solution, the Fe source can be added in form of oxide Fe₂O₃. The Fe source is preferably selected from iron oxide red and/or iron oxide yellow, more preferably a composition of iron oxide red and iron oxide yellow, wherein the mass ratio of iron oxide red to iron oxide yellow as Fe₂O₃ can be 1.0-3.5:1. The first K source can be added in form of potassium salt; the potassium salt can be any one or more of potassium carbonate, potassium nitrate, and potassium bicarbonate. The Ce source can be added in form of cerium salt; and the cerium salt can be cerium nitrate. The Mo source can be added in form of molybdenum salt or oxide; and the molybdenum salt can be ammonium molybdate. The calcium source can be added in form of oxide or hydroxide. The pore-forming agent can be any one or more of activated carbon, graphite, sodium carboxymethylcellulose and polystyrene microsphere. The pore-forming agent can be added in an amount of 0-5% by mass of the catalyst. In the preparation method of the catalyst, the Fe source, the Ce source, the Mo source, the Ca source, the first K source, and the pore-forming agent can be all added in form of solid phase powder.

In the above-mentioned technical solution, a raw material may further contain a Ti source; the Ti source together with the Fe source, the Ce source, the Mo source, the Ca source, optionally the first K source, and optionally the pore-forming agent can be mixed evenly and added. The Ti source may be added in form of titanium salt or oxide; and the titanium salt may be any one or both of titanium tetrachloride or titanium tetrabromide.

In the above-mentioned technical solution, the mixing can be carried out using conventional mechanical stirring methods.

In the above-mentioned technical solution, the condition for letting stand for reaction can comprise letting stand for reaction at 120-180°C for 12-48 hours. The container for letting stand for reaction is preferably an autoclave. The pressure for letting stand for reaction is not particularly limited, for example, 2-20 atm.

In the above-mentioned technical solution, the shaping can be extrusion shaping, and the shaped bodies can be particles with a diameter of 2-5 mm and a length of 3-10 mm. If the material after the reaction cannot meet the shaping requirements, a part of the moisture can be removed by evaporation or other methods or an appropriate amount of moisture can be added before shaping.

In the above-mentioned technical solution, the temperature of the calcining can be 600-1000°C, and the time of the calcining can be 2-8 hours.

In the above-mentioned technical solution, as a preferable calcining condition, a two-step calcining is used, for example but not limited to calcining at 600-800°C for 2-4 hours, then calcining at 900-1000°C for 2-4 hours. The calcining can be carried out in a muffle furnace.

In the above-mentioned technical solution, a shaped material can be firstly dried and then calcined. The drying temperature can be 50-200°C, the drying time can be 1-24 hours.

In order to solve the third technical problem, the third aspect of the present invention provides use of the above-mentioned ethylbenzene dehydrogenation catalyst in the production of styrene by ethylbenzene dehydrogenation.

In the above-mentioned technical solution, the use of the ethylbenzene dehydrogenation catalyst in the production of styrene is suitable for the ethylbenzene dehydrogenation at a steam/ethylbenzene ratio of 2.0 or less, preferably 1.3-2.0.

In the above-mentioned technical solution, the use of the ethylbenzene dehydrogenation catalyst in the production of styrene is suitable for the ethylbenzene dehydrogenation at a low steam/ethylbenzene ratio; and the low steam/ethylbenzene ratio is 1.3 or less, preferably 0.7-1.3.

In the above-mentioned technical solution, said use comprises: a raw material gas containing ethylbenzene contacts with the above-mentioned catalyst of the present invention in the presence of water vapor to perform a dehydrogenation reaction to produce a styrene-containing product.

In the above-mentioned technical solution, water can be pre-heated into water vapor before entering the reactor and thoroughly mixed with the raw material gas.

In the above-mentioned technical solution, the temperature of the dehydrogenation reaction can be 570-640°C.

In the above-mentioned technical solution, the pressure of the dehydrogenation reaction can be an absolute pressure of 20-100kPa.

In the above-mentioned technical solution, the weight hourly space velocity of ethylbenzene can be 0.2-2.0 h⁻¹.

In addition, this disclosure also includes the following technical solutions:
1. A catalyst, characterized in that the catalyst contains Fe, K, Ce, Mo, and Ca, and optionally Na; wherein the area of CeO₂ (100) exposed crystal face comprises 43% or higher, for example 50% or higher, preferably 60% or higher, more preferably 70% or higher, e.g., 70%, 75%, 80%, 85%, 87%, 90%, 92%, 95%, 97%, 99% and the like of the area of CeO₂ total exposed crystal face.
   Among others, the area of the exposed crystal surface is measured by a characterization means using a high-angle annular dark-field scanning transmission electron microscopy (HAADF-STEM). For example, the measuring instrument is the Titan Cubed Themis G2 300 transmission electron microscope with double spherical aberration correction of FEI Company;
   The ratio of the area of CeO₂ (100) exposed crystal face to the area of CeO₂ total exposed crystal face of the catalyst sample is obtained by collection and calculation based on the HAADF-STEM morphology photos and geometric structure characteristics of the catalyst, wherein at least 100 HAADF-STEM morphology photos are collected; for each photo, the area of each of exposed crystal faces of CeO₂ is acquired, the area of the maximal exposed crystal face is taken as the basic reference, one or more crystal faces that have "an area of the exposed crystal face" higher than "0.001×the area of the maximal exposed crystal face" is/are chosen, the total area of said one or more exposed crystal faces is used as the area of CeO₂ total exposed crystal face, and then the ratio is calculated; and the ratios are averaged to be used as the ratio of the area of CeO₂ (100) exposed crystal face to the area of CeO₂ total exposed crystal face of the catalyst sample.
   For example, when observing a piece of HAADF-STEM morphology photo of a catalyst sample, it is observed that CeO₂ (100) has an area of the maximally exposed crystal face. Besides CeO₂ (100), only each of CeO₂(110), CeO₂(111), and CeO₂(311) has an area of the exposed crystal face greater than [0.001×the area of the exposed crystal face of CeO₂(100)], the total area of the exposed crystal faces of CeO₂(100), CeO₂(110), CeO₂(111), and CeO₂(311) is taken as the area of CeO₂ total exposed crystal face, and the ratio is calculated to obtain the ratio of the area of CeO₂ (100) exposed crystal face to the area of CeO₂ total exposed crystal face for this morphology photo; in this way, at least 100 HAADF-STEM morphology photos of the catalyst sample are collected and calculated, and the obtained average value is taken as the ratio of the area of CeO₂ (100) exposed crystal face to the area of CeO₂ total exposed crystal face for the catalyst sample.
2. The catalyst of technical solution 1, wherein Fe and K exist in the form of (K₂O)x(Fe₂O₃)y, where x:y=1:1-1:11.
3. A catalyst, characterized in that the composition of the catalyst is calculated as oxide, the catalyst contains Fe₂O₃, K₂O, CeO₂, MoO₃, and CaO; wherein the area of CeO₂ (100) exposed crystal face comprises 43% or higher, for instance 50% or higher, preferably 60% or higher, more preferably 70% or higher of the area of CeO₂ total exposed crystal face.
4. The catalyst according to any one of the preceding technical solutions, characterized in that the H₂-TPR main reduction peak position of the catalyst is 570-620°C, such as 570°C, 580°C, 585°C, 590°C, 595°C, 600°C, 605°C, 610°C, 615°C, 620°C.
5. The catalyst according to any one of the preceding technical solutions, characterized in that the catalyst includes the following components in mass fraction based on the total mass of the catalyst:
   (a) 60%-85% of Fe₂O₃;
   (b) 6%-14% of K₂O;
   (c) 6%-14% of CeO₂;
   (d) 0.5%-5% of MoO₃;
   (e) 0.3%-7% of CaO.
6. The catalyst according to technical solution 5, characterized in that the catalyst, based on the total mass of the catalyst, in mass fraction, contains 0.01%-2.0% Na₂O.
7. The catalyst according to technical solution 5, characterized in that the catalyst, based on the total mass of the catalyst, in mass fraction, contains 0.01%-2.0% Na₂O, the catalyst further contains 0.01%-2.0% other metal oxide(s), such as TiO₂.
8. The catalyst according to any one of the preceding technical solutions, characterized in that the catalyst is used in the ethylbenzene dehydrogenation, preferably the ethylbenzene dehydrogenation in a low steam/ethylbenzene ratio.
9. A method for preparing the catalyst according to any one of the preceding technical solutions, characterized in that said method comprises the following steps:
   mixing a Fe source, a Ce source, a Mo source, a Ca source, optionally a first K source, and optionally a pore-forming agent evenly, then adding an alkaline solution, letting stand for reaction, shaping, and calcining to produce the catalyst;
   wherein, the K₂O in the catalyst derives from the first K source and/or the alkaline solution, for example, at least 50% of the K₂O in the catalyst derives from an alkaline solution containing K, and the remaining part derives from the first K source; or the K₂O in the catalyst can also all derive from an alkaline solution containing K instead of using the first K source.
10. The preparation method according to any one of the preceding technical solutions, characterized in that the alkaline solution is an aqueous solution of potassium hydroxide and/or sodium hydroxide, preferably an aqueous solution of sodium hydroxide and potassium hydroxide, the mass ratio of sodium hydroxide as Na₂O to potassium hydroxide as K₂O is preferably 1:2-23; the OH group concentration of the alkaline solution is 1 mol/L - 8 mol/L;
   and/or, the alkaline solution is an aqueous solution of sodium hydroxide and potassium hydroxide, the mass ratio of sodium hydroxide as Na₂O to potassium hydroxide as K₂O is preferably 1:2-23.
11. The preparation method according to any one of the preceding technical solutions, characterized in that at leaat a part of the K₂O in the catalyst derives from the alkaline solution; preferably, at least 50% of the K₂O in the catalyst derives from an alkaline solution containing K, the remaining part derives from the first K source.
12. The preparation method according to any one of the preceding technical solutions, characterized in that a raw material further contains a Ti source; the Ti source together with the Fe source, the Ce source, the Mo source, the Ca source, optionally the first K source, and optionally the pore-forming agent is mixed evenly and added; the Ti source is added in form of titanium salt or oxide; preferably, the titanium salt is any one or both of titanium tetrachloride or titanium tetrabromide.
13. The preparation method according to any one of the preceding technical solutions, characterized in that the condition for letting stand for reaction comprises letting stand for reaction at 120-180°C for 12-48 hours.
14. The preparation method according to any one of the preceding technical solutions, characterized in that the calcining temperature is 600-1000°C, the calcining time is 2-8 hours;
   for example a two-step calcining is used, for example but not limited to calcining at 600-800°C for 2-4 hours, then calcining at 900-1000°C for 2-4 hours.
15. The preparation method according to any one of the preceding technical solutions, characterized in that the calcining is carried out in a muffle furnace.
16. The preparation method according to any one of the preceding technical solutions, characterized in that a shaped material is firstly dried and then calcined, the drying temperature is 50-200°C, the drying time is 1-24 hours.
17. The preparation method according to any one of the preceding technical solutions, characterized in that the reaction is carried out under an increased pressure, for example 2-20 atm (gauge pressure).
18. The preparation method according to any one of the preceding technical solutions, characterized in that the Ce source is added in form of cerium salt, for example the Ce source is cerium nitrate;
   and/or, the Fe source is added in form of oxide Fe₂O₃, for example the Fe source is preferably selected from iron oxide red and/or iron oxide yellow, more preferably a composition of iron oxide red and iron oxide yellow, wherein the mass ratio of iron oxide red to iron oxide yellow as Fe₂O₃ is 1.0-3.5:1; and/or, the first K source is added in form of potassium salt; the potassium salt is any one or more of potassium carbonate, potassium nitrate, and potassium bicarbonate;
   and/or, the Mo source is added in form of molybdenum salt or oxide; and the molybdenum salt is ammonium molybdate;
   and/or, the calcium source is added in form of oxide or hydroxide;
   optionally, the pore-forming agent is any one or more of activated carbon, graphite, sodium carboxymethylcellulose and polystyrene microsphere, and the pore-forming agent is added in an amount of 0-5% of the catalyst mass;
   optionally, in the preparation method of the catalyst, the Fe source, the Ce source, the Mo source, the Ca source, the first K source, and the pore-forming agent are all added in form of solid phase powder.
19. Use of the catalyst according to any one of the preceding technical solutions or the catalyst prepared with the preparation method according to any one of the preceding technical solutions for producing styrene in the ethylbenzene dehydrogenation.
20. Use according to the preceding technical solution 19, characterized in that the catalyst is suitable for the ethylbenzene dehydrogenation in a low steam/ethylbenzene ratio; the low steam/ethylbenzene ratio is 1.3 or less, preferably 0.7-1.3.
21. Use according to the preceding technical solution 19, characterized in that the catalyst is suitable for the production of styrene with a steam/ethylbenzene ratio of 2.0 or less.
22. Use according to any one of the preceding technical solutions, characterized in that said use comprises: a raw material gas containing ethylbenzene contacts with the catalyst in the presence of water vapor to perform a dehydrogenation reaction to produce a styrene-containing product;
   water is pre-heated into water vapor before entering the reactor and thoroughly mixed with the raw material gas;
   the temperature of the dehydrogenation reaction is 570-640°C;
   the pressure of the dehydrogenation reaction is an absolute pressure of 20-100kPa;
   the weight hourly space velocity of ethylbenzene is 0.2-2.0 h⁻¹.

Compared with the existing technology, the present invention has significant advantages and outstanding effects, specifically as follows:
1. During the ethylbenzene dehydrogenation, the surface of the catalyst is covered with H after capturing H in the ethylbenzene molecule, and the surface is reduced. It is necessary to remove H₂ to restore the reaction activity. The active phase potassium ferrite in the Fe-K-Ce-Mo-type catalyst has a low rate for removing the surface H₂, which limits the catalyst reaction activity. H on the surface of the active phase of the catalyst can migrate to the CeO₂ surface with the promotion of water, quickly forming H₂ and then leaving the catalyst surface, which reduces the reaction energy barrier, promotes the reaction, and improves the catalytic activity of the catalyst. The inventors found through research that the reaction performance of the Fe-K-Ce-Mo-type ethylbenzene dehydrogenation catalyst is highly related to its surface structure. Different exposed crystal faces of CeO₂ will lead to significant differences in the dehydrogenation activity and stability of the catalyst. After further research, the inventors found that the higher ratio of the area of CeO₂ (100) exposed crystal face,especially more than 60%, promotes the reoxidation of the active sites of the catalyst in the ethylbenzene dehydrogenation, and the reduction temperature of the catalyst is increased by cooperating with auxiliaries so that the catalyst has good activity and good stability.
2. In the present invention, the inventors found through research that during the preparation of the Fe-K-Ce-Mo-type ethylbenzene dehydrogenation catalyst, by controlling the alkalinity of the system and the wet reaction conditions, on the one hand, the surface of the prepared catalyst has a high proportion of the CeO₂(100) exposed crystal face. On the other hand, this preparation method promotes the interaction between the auxiliary component and the active component, improving the stability of the crystal structure in the catalyst. Compared with conventional dry process, it is more conducive to improving the catalytic performance of ethylbenzene dehydrogenation catalyst. The preparation method of the catalyst is simple, and the obtained catalyst has the advantages of high activity, high styrene selectivity, and the performance of the catalyst remains stable after 1,000 hours of operation.
3. The catalyst of the present invention is used in the reaction of ethylbenzene dehydrogenation to prepare styrene. It has high activity, high selectivity and high stability under low to high steam/ethylbenzene ratio, especially low steam/ethylbenzene ratio, and has achieved good technical effects.

Using the technical solution of the present invention, the activity of the catalyst of the present invention is evaluated in an isothermal fixed bed at an absolute pressure of 70 kPa, a liquid hourly space velocity of 1.0 h⁻¹ , at 620°C, under a steam/ethylbenzene ratio of 0.8 (wt) to which the usually used ratio of 2.0 (wt) was reduced, and with a conversion rate of >75%. After 1,000 hours of operation, the catalyst activity remains stable and the conversion rate does not change significantly. It shows that the catalyst of the present invention, when used in the reaction of ethylbenzene dehydrogenation to prepare styrene, especially at low steam/ethylbenzene ratio, has high activity and stability, and achieves good technical effects.

### Description of the drawings

Figure 1 is a photo of the main exposed crystal face in the HAADF-STEM test of the catalyst of Example 1;
Figure 2 is a photo of the main exposed crystal face in the HAADF-STEM test of the catalyst of Comparative Example 1;
Figure 3 is the H₂-TPR diagram of the catalysts of Example 1 and Comparative Example 1.

### Detailed description

The present invention will be further described below through examples, but the protection scope of the present invention is not limited by the examples.

In the present invention, the area of the exposed crystal surface is measured by a characterization means using a high-angle annular dark-field scanning transmission electron microscopy (HAADF-STEM), and the measuring instrument is the Titan Cubed Themis G2 300 transmission electron microscope with double spherical aberration correction of FEI Company. The ratio of the area of CeO₂ (100) exposed crystal face to the area of CeO₂ total exposed crystal face is obtained by collecting HAADF-STEM morphology photo of a catalyst sample and calculation, i.e. it is a ratio of the observed area of the CeO₂ (100) crystal face to the observed area of CeO₂ total exposed crystal face. The area of CeO₂ total exposed crystal face is the total area of the exposed crystal faces CeO₂ (100), CeO₂ (110), CeO₂ (111) and CeO₂ (311). By observing the exposed crystal face of CeO₂ (100) with a scanning transmission electron microscope, two mutually perpendicular directions [020] and [002] can be seen in the plane perpendicular to the [100] observation direction, and their interplanar distances are both 260-290pm, proving the exposed crystal face is CeO₂(100).

In the present invention, the H₂-TPR experiment of the catalyst is carried out with an AutoChem II 2920 chemical adsorption instrument. 0.1g of the catalyst is weighed and placed in a U-shaped quartz reaction tube. It is first purged with a 30mL/min high-purity Ar gas flow at 200°C for 2 hours. Then hydrogen gas is used as the reducing gas, and argon gas is used as the balance gas. A mixed gas of 10% H₂/Ar is introduced at a rate of 30mL/min. The system is heated to a certain temperature at 10K/min. The H₂ consumption during the heating process is recorded with a TCD detector. The H₂-TPR main reduction peak position is the temperature position corresponding to the most intense peak of the signals in the spectrum.

In the present invention, the catalyst of the present invention is evaluated for the ethylbenzene dehydrogenation performance in an isothermal fixed bed. The process is briefly described as follows: The reactor is a stainless steel tube with an inner diameter of 1", filled with 50-150 mL of catalyst with a diameter of 3-10 mm. Deionized water and ethylbenzene are respectively input into a preheating mixer through a metering pump. They are preheated and mixed into a gaseous state before entering the reactor. The reactor is heated by an electric heating wire to reach a predetermined temperature. The reaction substance leaving the reactor is condensed by water and analyzed by gas chromatography.

The ethylbenzene conversion rate and the styrene selectivity are calculated according to the following equations: Ethylbenzene conversion rate (%) = [ethylbenzene concentration before the reaction (wt%) - ethylbenzene concentration after the reaction (wt%)]/ethylbenzene concentration before the reaction (wt%)×100%, Styrene selectivity (%) = the concentration of the formed styrene (wt%)/[ethylbenzene concentration before the reaction (wt%) - ethylbenzene concentration after the reaction (wt%)]×100%.

### [Example 1]

Iron oxide red equivalent to 50.1 parts of Fe₂O₃, iron oxide yellow equivalent to 21.4 parts of Fe₂O₃, cerium nitrate equivalent to 10.5 parts of CeO₂, ammonium molybdate equivalent to 2.8 parts of MoO₃, 3.2 parts of calcium oxide and 3.05 parts of polystyrene microspheres were weighed and added to a mixer, and the resulting mixture was stirred for 2 hours until evenly mixed. Then a mixed aqueous solution of NaOH equivalent to 0.8 parts of Na₂O and KOH equivalent to 11.2 parts of K₂O was added, and the OH concentration in the mixed aqueous solution was 3.2 mol/L. The mixture was left in the autoclave to stand for reacting at 140°C for 20 hrs. Then, after adjusting the water content, the above mixture was extruded and pelletized to obtain pellets with a diameter of 3 mm and a length of 6 mm, which were placed in an oven and dried at 80°C for 4 hrs and 150°C for 4 hrs, then placed in a muffle furnace and calcined at 650°C for 2 hrs and at 900°C for 2 hrs to obtain a finished catalyst. The composition of the catalyst is shown in Table 1.

The presence of K and Fe in the catalyst of Example 1 in the ratio of K_{1.81}Fe_{10.73}O₁₇ was determined based on the XRD crystal phase analysis.

100 mL of the catalyst was put into a reactor for performance evaluation under the conditions of an absolute pressure of 70 kPa, a liquid hourly space velocity of 1.0 h⁻¹, a temperature of 620°C, and a steam/ethylbenzene ratio of 0.8 (wt). The test results after the 100-hours reaction are listed in Table 1, and the test results after the 1000-hours reaction are listed in Table 2.

### [Example 2]

Iron oxide red equivalent to 36.2 parts of Fe₂O₃, iron oxide yellow equivalent to 26.6 parts of Fe₂O₃, cerium nitrate equivalent to 11.6 parts of CeO₂, ammonium molybdate equivalent to 4.8 parts of MoO₃, 5.4 parts of calcium oxide and 2.6 parts of polystyrene microspheres were weighed and added to a mixer, and the resulting mixture was stirred for 2 hours until evenly mixed. Then a mixed aqueous solution of NaOH equivalent to 1.8 parts of Na₂O and KOH equivalent to 13.6 parts of K₂O was added, and the OH concentration in the mixed aqueous solution was 6.5 mol/L. The mixture was left in the autoclave to stand for reacting at 120°C for 48 hrs. Then, after adjusting the water content, the above mixture was extruded and pelletized to obtain pellets with a diameter of 3 mm and a length of 6 mm, which were placed in an oven and dried at 80°C for 4 hrs and 150°C for 4 hrs, then placed in a muffle furnace and calcined at 650°C for 2 hrs and at 900°C for 2 hrs to obtain a finished catalyst. The composition of the catalyst is shown in Table 1.

100 mL of the catalyst was put into a reactor for performance evaluation under the conditions of an absolute pressure of 70 kPa, a liquid hourly space velocity of 1.0 h⁻¹, a temperature of 620°C, and a steam/ethylbenzene ratio of 0.8 (wt). The test results after the 100-hours reaction are listed in Table 1.

### [Example 3]

Iron oxide red equivalent to 45.6 parts of Fe₂O₃, iron oxide yellow equivalent to 23.8 parts of Fe₂O₃, cerium nitrate equivalent to 13.8 parts of CeO₂, ammonium molybdate equivalent to 3.6 parts of MoO₃ and 1.5 parts of calcium oxide were weighed and added to a mixer, and the resulting mixture was stirred for 2 hours until evenly mixed. Then a mixed aqueous solution of NaOH equivalent to 0.9 parts of Na₂O and KOH equivalent to 10.8 parts of K₂O was added, and the OH concentration in the mixed aqueous solution was 2.5 mol/L. The mixture was left in the autoclave to stand for reacting at 170°C for 12 hrs. Then, after adjusting the water content, the above mixture was extruded and pelletized to obtain pellets with a diameter of 3 mm and a length of 6 mm, which were placed in an oven and dried at 80°C for 4 hrs and 150°C for 4 hrs, then placed in a muffle furnace and calcined at 650°C for 2 hrs and at 900°C for 2 hrs to obtain a finished catalyst. The composition of the catalyst is shown in Table 1.

100 mL of the catalyst was put into a reactor for performance evaluation under the conditions of an absolute pressure of 70 kPa, a liquid hourly space velocity of 1.0 h⁻¹, a temperature of 620°C, and a steam/ethylbenzene ratio of 0.8 (wt). The test results after the 100-hours reaction are listed in Table 1.

### [Example 4]

Iron oxide red equivalent to 48.2 parts of Fe₂O₃, iron oxide yellow equivalent to 34.9 parts of Fe₂O₃, cerium nitrate equivalent to 8.3 parts of CeO₂, ammonium molybdate equivalent to 1.2 parts of MoO₃, 0.4 parts of calcium oxide and 4.8 parts of polystyrene microspheres were weighed and added to a mixer, and the resulting mixture was stirred for 2 hours until evenly mixed. Then a mixed aqueous solution of NaOH equivalent to 0.8 parts of Na₂O and KOH equivalent to 6.2 parts of K₂O was added, and the OH concentration in the mixed aqueous solution was 2.0 mol/L. The mixture was left in the autoclave to stand for reacting at 140°C for 20 hrs. Then, after adjusting the water content, the above mixture was extruded and pelletized to obtain pellets with a diameter of 3 mm and a length of 6 mm, which were placed in an oven and dried at 80°C for 4 hrs and 150°C for 4 hrs, then placed in a muffle furnace and calcined at 600°C for 4 hrs and at 900°C for 2 hrs to obtain a finished catalyst. The composition of the catalyst is shown in Table 1.

100 mL of the catalyst was put into a reactor for performance evaluation under the conditions of an absolute pressure of 70 kPa, a liquid hourly space velocity of 1.0 h⁻¹, a temperature of 620°C, and a steam/ethylbenzene ratio of 0.8 (wt). The test results after the 100-hours reaction are listed in Table 1.

### [Example 5]

Iron oxide red equivalent to 52.3 parts of Fe₂O₃, iron oxide yellow equivalent to 28.0 parts of Fe₂O₃, cerium nitrate equivalent to 8.1 parts of CeO₂, ammonium molybdate equivalent to 0.6 parts of MoO₃, 2.6 parts of calcium oxide and 2.5 parts of polystyrene microspheres were weighed and added to a mixer, and the resulting mixture was stirred for 2 hours until evenly mixed. Then a mixed aqueous solution of NaOH equivalent to 1.0 parts of Na₂O and KOH equivalent to 7.4 parts of K₂O was added, and the OH concentration in the mixed aqueous solution was 2.6 mol/L. The mixture was left in the autoclave to stand for reacting at 140°C for 20 hrs. Then, after adjusting the water content, the above mixture was extruded and pelletized to obtain pellets with a diameter of 3 mm and a length of 6 mm, which were placed in an oven and dried at 80°C for 4 hrs and 150°C for 4 hrs, then placed in a muffle furnace and calcined at 750°C for 2 hrs and at 900°C for 2 hrs to obtain a finished catalyst. The composition of the catalyst is shown in Table 1.

100 mL of the catalyst was put into a reactor for performance evaluation under the conditions of an absolute pressure of 70 kPa, a liquid hourly space velocity of 1.0 h⁻¹, a temperature of 620°C, and a steam/ethylbenzene ratio of 0.8 (wt). The test results after the 100-hours reaction are listed in Table 1.

### [Example 6]

Iron oxide red equivalent to 50.6 parts of Fe₂O₃, iron oxide yellow equivalent to 20.3 parts of Fe₂O₃, cerium nitrate equivalent to 6.9 parts of CeO₂, ammonium molybdate equivalent to 3.5 parts of MoO₃, 6.9 parts of calcium oxide, 0.06 parts of titanium oxide and 2.6 parts of polystyrene microspheres were weighed and added to a mixer, and the resulting mixture was stirred for 2 hours until evenly mixed. Then a mixed aqueous solution of NaOH equivalent to 0.6 parts of Na₂O and KOH equivalent to 11.14 parts of K₂O was added, and the OH concentration in the mixed aqueous solution was 3.1 mol/L. The mixture was left in the autoclave to stand for reacting at 140°C for 20 hrs. Then, after adjusting the water content, the above mixture was extruded and pelletized to obtain pellets with a diameter of 3 mm and a length of 6 mm, which were placed in an oven and dried at 80°C for 4 hrs and 150°C for 4 hrs, then placed in a muffle furnace and calcined at 650°C for 2 hrs and at 980°C for 2 hrs to obtain a finished catalyst. The composition of the catalyst is shown in Table 1.

100 mL of the catalyst was put into a reactor for performance evaluation under the conditions of an absolute pressure of 70 kPa, a liquid hourly space velocity of 1.0 h⁻¹, a temperature of 620°C, and a steam/ethylbenzene ratio of 0.8 (wt). The test results after the 100-hours reaction are listed in Table 1.

### [Example 7]

Iron oxide red equivalent to 54.4 parts of Fe₂O₃, iron oxide yellow equivalent to 16.0 parts of Fe₂O₃, cerium nitrate equivalent to 11.2 parts of CeO₂, 3.0 parts of MoO₃, calcium carbonate equivalent to 3.2 parts of CaO and 3.1 parts of polystyrene microspheres were weighed and added to a mixer, and the resulting mixture was stirred for 2 hours until evenly mixed. Then a mixed aqueous solution of NaOH equivalent to 1.6 parts of Na₂O and KOH equivalent to 10.6 parts of K₂O was added, and the OH concentration in the mixed aqueous solution was 3.0 mol/L. The mixture was left in the autoclave to stand for reacting at 140°C for 20 hrs. Then, after adjusting the water content, the above mixture was extruded and pelletized to obtain pellets with a diameter of 3 mm and a length of 6 mm, which were placed in an oven and dried at 50°C for 12 hrs and at 150°C for 4 hrs, then placed in a muffle furnace and calcined at 650°C for 2 hrs and at 900°C for 2 hrs to obtain a finished catalyst. The composition of the catalyst is shown in Table 1.

100 mL of the catalyst was put into a reactor for performance evaluation under the conditions of an absolute pressure of 70 kPa, a liquid hourly space velocity of 1.0 h⁻¹, a temperature of 620°C, and a steam/ethylbenzene ratio of 0.8 (wt). The test results after the 100-hours reaction are listed in Table 1. The test results after the 1000-hours reaction are listed in Table 2.

### [Example 8]

Iron oxide red equivalent to 37.5 parts of Fe₂O₃, iron oxide yellow equivalent to 35.1 parts of Fe₂O₃, cerium nitrate equivalent to 11.9 parts of CeO₂, ammonium molybdate equivalent to 2.1 parts of MoO₃, calcium nitrate equivalent to 3.3 parts of CaO and 3.5 parts of activated carbon were weighed and added to a mixer, and the resulting mixture was stirred for 2 hours until evenly mixed. Then a mixed aqueous solution of NaOH equivalent to 1.5 parts of Na₂O and KOH equivalent to 8.6 parts of K₂O was added, and the OH concentration in the mixed aqueous solution was 2.8 mol/L. The mixture was left in the autoclave to stand for reacting at 140°C for 20 hrs. Then, after adjusting the water content, the above mixture was extruded and pelletized to obtain pellets with a diameter of 3 mm and a length of 6 mm, which were placed in an oven and dried at 100°C for 4 hrs and at 150°C for 4 hrs, then placed in a muffle furnace and calcined at 650°C for 2 hrs and at 900°C for 2 hrs to obtain a finished catalyst.

The composition of the catalyst is shown in Table 1.

100 mL of the catalyst was put into a reactor for performance evaluation under the conditions of an absolute pressure of 70 kPa, a liquid hourly space velocity of 1.0 h⁻¹, a temperature of 620°C, and a steam/ethylbenzene ratio of 0.8 (wt). The test results after the 100-hours reaction are listed in Table 1.

### [Example 9]

Iron oxide red equivalent to 40.0 parts of Fe₂O₃, iron oxide yellow equivalent to 32.5 parts of Fe₂O₃, cerium nitrate equivalent to 10.6 parts of CeO₂, ammonium molybdate equivalent to 2.1 parts of MoO₃, calcium chloride equivalent to 2.9 parts of CaO and 3.2 parts of graphite were weighed and added to a mixer, and the resulting mixture was stirred for 2 hours until evenly mixed. Then a mixed aqueous solution of NaOH equivalent to 0.5 parts of Na₂O and KOH equivalent to 11.4 parts of K₂O was added, and the OH concentration in the mixed aqueous solution was 3.5 mol/L. The mixture was left in the autoclave to stand for reacting at 140°C for 20 hrs. Then, after adjusting the water content, the above mixture was extruded and pelletized to obtain pellets with a diameter of 3 mm and a length of 6 mm, which were placed in an oven and dried at 120°C for 4 hrs and at 150°C for 4 hrs, then placed in a muffle furnace and calcined at 650°C for 2 hrs and at 900°C for 2 hrs to obtain a finished catalyst. The composition of the catalyst is shown in Table 1.

100 mL of the catalyst was put into a reactor for performance evaluation under the conditions of an absolute pressure of 70 kPa, a liquid hourly space velocity of 1.0 h⁻¹, a temperature of 620°C, and a steam/ethylbenzene ratio of 0.8 (wt). The test results after the 100-hours reaction are listed in Table 1.

### [Example 10]

Iron oxide red equivalent to 50.5 parts of Fe₂O₃, iron oxide yellow equivalent to 19.7 parts of Fe₂O₃, cerium nitrate equivalent to 12.0 parts of CeO₂, 2.6 parts of MoO₃, 3.5 parts of calcium oxide and 3.2 parts of hydroxymethylcellulose were weighed and added to a mixer, and the resulting mixture was stirred for 2 hours until evenly mixed. Then a mixed aqueous solution of NaOH equivalent to 1.1 parts of Na₂O and KOH equivalent to 10.6 parts of K₂O was added, and the OH concentration in the mixed aqueous solution was 3.4 mol/L. The mixture was left in the autoclave to stand for reacting at 140°C for 20 hrs. Then, after adjusting the water content, the above mixture was extruded and pelletized to obtain pellets with a diameter of 3 mm and a length of 6 mm, which were placed in an oven and dried at 80°C for 4 hrs and at 180°C for 4 hrs, then placed in a muffle furnace and calcined at 650°C for 2 hrs and at 900°C for 2 hrs to obtain a finished catalyst. The composition of the catalyst is shown in Table 1.

100 mL of the catalyst was put into a reactor for performance evaluation under the conditions of an absolute pressure of 70 kPa, a liquid hourly space velocity of 1.0 h⁻¹, a temperature of 620°C, and a steam/ethylbenzene ratio of 0.8 (wt). The test results after the 100-hours reaction are listed in Table 1.

### [Example 11]

Iron oxide red equivalent to 50.9 parts of Fe₂O₃, iron oxide yellow equivalent to 23.4 parts of Fe₂O₃, cerium nitrate equivalent to 11.2 parts of CeO₂, ammonium molybdate equivalent to 1.8 parts of MoO₃, calcium carbonate equivalent to 2.7 parts of CaO and 3.2 parts of graphite were weighed and added to a mixer, and the resulting mixture was stirred for 2 hours until evenly mixed. Then a mixed aqueous solution of NaOH equivalent to 0.5 parts of Na₂O and KOH equivalent to 9.5 parts of K₂O was added, and the OH concentration in the mixed aqueous solution was 2.9 mol/L. The mixture was left in the autoclave to stand for reacting at 140°C for 20 hrs. Then, after adjusting the water content, the above mixture was extruded and pelletized to obtain pellets with a diameter of 3 mm and a length of 6 mm, which were placed in an oven and dried at 80°C for 4 hrs and at 200°C for 2 hrs, then placed in a muffle furnace and calcined at 650°C for 2 hrs and at 900°C for 2 hrs to obtain a finished catalyst. The composition of the catalyst is shown in Table 1.

100 mL of the catalyst was put into a reactor for performance evaluation under the conditions of an absolute pressure of 70 kPa, a liquid hourly space velocity of 1.0 h⁻¹, a temperature of 620°C, and a steam/ethylbenzene ratio of 0.8 (wt). The test results after the 100-hours reaction are listed in Table 1.

### [Example 12]

Iron oxide red equivalent to 50.1 parts of Fe₂O₃, iron oxide yellow equivalent to 21.4 parts of Fe₂O₃, cerium nitrate equivalent to 10.5 parts of CeO₂, potassium nitrate equivalent to 9.6 parts of K₂O, ammonium molybdate equivalent to 2.8 parts of MoO₃, 3.2 parts of calcium oxide and 3.05 parts of polystyrene microspheres were weighed and added to a mixer, and the resulting mixture was stirred for 2 hours until evenly mixed. Then a mixed aqueous solution of NaOH equivalent to 0.8 parts of Na₂O and KOH equivalent to 1.6 parts of K₂O was added, and the OH concentration in the mixed aqueous solution was 3.2 mol/L. The mixture was left in the autoclave to stand for reacting at 140°C for 20 hrs. Then, after adjusting the water content, the above mixture was extruded and pelletized to obtain pellets with a diameter of 3 mm and a length of 6 mm, which were placed in an oven and dried at 80°C for 4 hrs and 150°C for 4 hrs, then placed in a muffle furnace and calcined at 650°C for 2 hrs and at 900°C for 2 hrs to obtain a finished catalyst. The composition of the catalyst is shown in Table 1.

100 mL of the catalyst was put into a reactor for performance evaluation under the conditions of an absolute pressure of 70 kPa, a liquid hourly space velocity of 1.0 h⁻¹, a temperature of 620°C, and a steam/ethylbenzene ratio of 0.8 (wt). The test results after the 100-hours reaction are listed in Table 1. The test results after the 1000-hours reaction are listed in Table 2.

### [Example 13]

Iron oxide red equivalent to 50.1 parts of Fe₂O₃, iron oxide yellow equivalent to 21.4 parts of Fe₂O₃, cerium nitrate equivalent to 10.5 parts of CeO₂, potassium carbonate equivalent to 11.2 parts of K₂O, ammonium molybdate equivalent to 2.8 parts of MoO₃, 3.2 parts of calcium oxide and 3.05 parts of polystyrene microspheres were weighed and added to a mixer, and the resulting mixture was stirred for 2 hours until evenly mixed. Then an aqueous solution of NaOH equivalent to 0.8 parts of Na₂O was added, and the OH concentration in the aqueous solution was 3.2 mol/L. The mixture was left in the autoclave to stand for reacting at 140°C for 20 hrs. Then, after adjusting the water content, the above mixture was extruded and pelletized to obtain pellets with a diameter of 3 mm and a length of 6 mm, which were placed in an oven and dried at 80°C for 4 hrs and 150°C for 4 hrs, then placed in a muffle furnace and calcined at 650°C for 2 hrs and at 900°C for 2 hrs to obtain a finished catalyst. The composition of the catalyst is shown in Table 1.

100 mL of the catalyst was put into a reactor for performance evaluation under the conditions of an absolute pressure of 70 kPa, a liquid hourly space velocity of 1.0 h⁻¹, a temperature of 620°C, and a steam/ethylbenzene ratio of 0.8 (wt). The test results after the 100-hours reaction are listed in Table 1.

### [Example 14]

Iron oxide red equivalent to 50.1 parts of Fe₂O₃, iron oxide yellow equivalent to 21.4 parts of Fe₂O₃, cerium nitrate equivalent to 10.5 parts of CeO₂, ammonium molybdate equivalent to 2.8 parts of MoO₃, 3.2 parts of calcium oxide, sodium nitrate equivalent to 0.8 parts of Na₂O, and 3.05 parts of polystyrene microspheres were weighed and added to a mixer, and the resulting mixture was stirred for 2 hours until evenly mixed. Then an aqueous solution of KOH equivalent to 11.2 parts of K₂O was added, and the OH concentration in the aqueous solution was 3.2 mol/L. The mixture was left in the autoclave to stand for reacting at 140°C for 20 hrs. Then, after adjusting the water content, the above mixture was extruded and pelletized to obtain pellets with a diameter of 3 mm and a length of 6 mm, which were placed in an oven and dried at 80°C for 4 hrs and 150°C for 4 hrs, then placed in a muffle furnace and calcined at 650°C for 2 hrs and at 900°C for 2 hrs to obtain a finished catalyst. The composition of the catalyst is shown in Table 1.

100 mL of the catalyst was put into a reactor for performance evaluation under the conditions of an absolute pressure of 70 kPa, a liquid hourly space velocity of 1.0 h⁻¹, a temperature of 620°C, and a steam/ethylbenzene ratio of 0.8 (wt). The test results after the 100-hours reaction are listed in Table 1, and the test results after the 1000-hours reaction are listed in Table 2.

### [Example 15]

Iron oxide red equivalent to 50.1 parts of Fe₂O₃, iron oxide yellow equivalent to 21.4 parts of Fe₂O₃, cerium nitrate equivalent to 10.5 parts of CeO₂, ammonium molybdate equivalent to 2.8 parts of MoO₃, 3.2 parts of calcium oxide and 3.05 parts of polystyrene microspheres were weighed and added to a mixer, and the resulting mixture was stirred for 2 hours until evenly mixed. Then an aqueous solution of KOH equivalent to 11.2 parts of K₂O was added, and the OH concentration in the aqueous solution was 3.2 mol/L. The mixture was left in the autoclave to stand for reacting at 140°C for 20 hrs. Then, after adjusting the water content, the above mixture was extruded and pelletized to obtain pellets with a diameter of 3 mm and a length of 6 mm, which were placed in an oven and dried at 80°C for 4 hrs and 150°C for 4 hrs, then placed in a muffle furnace and calcined at 650°C for 2 hrs and at 900°C for 2 hrs to obtain a finished catalyst. The composition of the catalyst is shown in Table 1.

100 mL of the catalyst was put into a reactor for performance evaluation under the conditions of an absolute pressure of 70 kPa, a liquid hourly space velocity of 1.0 h⁻¹, a temperature of 620°C, and a steam/ethylbenzene ratio of 0.8 (wt). The test results after the 100-hours reaction are listed in Table 1.

### [Comparative Example 1]

Iron oxide red equivalent to 50.1 parts of Fe₂O₃, iron oxide yellow equivalent to 21.4 parts of Fe₂O₃, cerium nitrate equivalent to 10.5 parts of CeO₂, potassium carbonate equivalent to 11.2 parts of K₂O, ammonium molybdate equivalent to 2.8 parts of MoO₃, 4.0 parts of calcium oxide and 3.05 parts of polystyrene microspheres were weighed and added together with the same amount of water as in Example 1 to a mixer, and the resulting mixture was stirred for 2 hours until evenly mixed. The concentration of hydroxy groups in the mixed aqueous solution was 0.3mol/L. The mixture was left in the autoclave to stand at 140°C for 20 hrs. Then, the above mixture was extruded and pelletized to obtain pellets with a diameter of 3 mm and a length of 6 mm, which were placed in an oven and dried at 80°C for 4 hrs and 150°C for 4 hrs, then placed in a muffle furnace and calcined at 650°C for 2 hrs and at 900°C for 2 hrs to obtain a finished catalyst. The catalyst evaluation method was the same as that in Example 1. The test result and the catalyst composition are listed in Tables 1 and 2.

### [Comparative Example 2]

Iron oxide red equivalent to 39.6 parts of Fe₂O₃, iron oxide yellow equivalent to 21.4 parts of Fe₂O₃, cerium nitrate equivalent to 21.0 parts of CeO₂, ammonium molybdate equivalent to 2.8 parts of MoO₃, 3.2 parts of calcium oxide and 3.05 parts of polystyrene microspheres were weighed and added to a mixer, and the resulting mixture was stirred for 2 hours until evenly mixed. Then a mixed aqueous solution of NaOH equivalent to 0.8 parts of Na₂O and KOH equivalent to 11.2 parts of K₂O was added, and the OH concentration in the mixed aqueous solution was 3.2 mol/L. The mixture was left in the autoclave to stand for reacting at 140°C for 20 hrs. Then, after adjusting the water content, the above mixture was extruded and pelletized to obtain pellets with a diameter of 3 mm and a length of 6 mm, which were placed in an oven and dried at 80°C for 4 hrs and 150°C for 4 hrs, then placed in a muffle furnace and calcined at 650°C for 2 hrs and at 900°C for 2 hrs to obtain a finished catalyst. The composition of the catalyst is shown in Table 1.

100 mL of the catalyst was put into a reactor for performance evaluation under the conditions of an absolute pressure of 70 kPa, a liquid hourly space velocity of 1.0 h⁻¹, a temperature of 620°C, and a steam/ethylbenzene ratio of 0.8 (wt). The test results after the 100-hours reaction are listed in Table 1, and the test results after the 1000-hours reaction are listed in Table 2.

### [Comparative Example 3]

Iron oxide yellow equivalent to 71.5 parts of Fe₂O₃ was weighed and mixed with an aqueous solution of cerium nitrate equivalent to 10.5 parts of CeO₂, and then mixed with an aqueous solution of KOH equivalent to 11.2 parts of K₂O, and the OH concentration in the mixed aqueous solution was 1.5 mol/L. The resulting paste was dried at 150°C for 2 hours and then calcined at 850°C for 2 hours.

The calcined product was ground, and then mixed with an aqueous suspension of 2.8 parts of MoO₃, 3.2 parts of calcium oxide, sodium carbonate equivalent to 0.8 parts of Na₂O and 3.05 parts of polystyrene microspheres, extruded into strips and pelletized to obtain pellets with a diameter of 3 mm and a length of 6 mm, which were placed in an oven and dried at 80°C for 4 hrs and 150°C for 4 hrs, then placed in a muffle furnace and calcined at 650°C for 2 hrs and at 900°C for 2 hrs to obtain a finished catalyst. The composition of the catalyst is shown in Table 1.

100 mL of the catalyst was put into a reactor for performance evaluation under the conditions of an absolute pressure of 70 kPa, a liquid hourly space velocity of 1.0 h⁻¹, a temperature of 620°C, and a steam/ethylbenzene ratio of 0.8 (wt). The test results after the 100-hours reaction are listed in Table 1, and the test results after the 1000-hours reaction are listed in Table 2.

### [Comparative Example 4]

Iron oxide red equivalent to 50.1 parts of Fe₂O₃, iron oxide yellow equivalent to 21.4 parts of Fe₂O₃, cerium nitrate equivalent to 10.5 parts of CeO₂, potassium carbonate equivalent to 10.64 parts of K₂O, NaOH equivalent to 0.8 parts of Na₂O, ammonium molybdate equivalent to 2.8 parts of MoO₃, 3.2 parts of calcium oxide and 3.05 parts of polystyrene microspheres were weighed and added to a mixer, and the resulting mixture was stirred for 2 hours until evenly mixed. Then deionized water was added, the resulting mixture was stirred for another 0.5 hours, extruded and pelletized to obtain pellets with a diameter of 3 mm and a length of 6 mm, which were placed in an oven and dried at 80°C for 4 hrs and 150°C for 4 hrs, then placed in a muffle furnace and calcined at 800°C for 4 hrs to produce a semi-finished catalyst. Then potassium carbonate equivalent to 0.56 parts of K₂O was dissolved in water, the semi-finished catalyst was impregnated in this solution with the OH concentration of 0.1 mol/L for 2 hours, and then placed in an oven and dried at 80°C for 4 hrs and 150°C for 4 hrs, then placed in a muffle furnace and calcined at 650°C for 2 hrs and at 900°C for 2 hrs to obtain a finished catalyst. The composition of the catalyst is shown in Table 1.

100 mL of the catalyst was put into a reactor for performance evaluation under the conditions of an absolute pressure of 70 kPa, a liquid hourly space velocity of 1.0 h⁻¹, a temperature of 620°C, and a steam/ethylbenzene ratio of 0.8 (wt). The test results after the 100-hours reaction are listed in Table 1, and the test results after the 1000-hours reaction are listed in Table 2.

### [Comparative Example 5]

Iron oxide red equivalent to 47.1 parts of Fe₂O₃, iron oxide yellow equivalent to 21.4 parts of Fe₂O₃, cerium nitrate equivalent to 10.5 parts of CeO₂, praseodymium nitrate equivalent to 3.0 parts of Pr₂O₃, ammonium molybdate equivalent to 2.8 parts of MoO₃, 3.2 parts of calcium oxide and 3.05 parts of polystyrene microspheres were weighed and added to a mixer, and the resulting mixture was stirred for 2 hours until evenly mixed. Then a mixed aqueous solution of NaOH equivalent to 0.8 parts of Na₂O and KOH equivalent to 11.2 parts of K₂O was added, and the OH concentration in the mixed aqueous solution was 3.2 mol/L. The mixture was left in the autoclave to stand for reacting at 140°C for 20 hrs. Then, after adjusting the water content, the above mixture was extruded and pelletized to obtain pellets with a diameter of 3 mm and a length of 6 mm, which were placed in an oven and dried at 80°C for 4 hrs and 150°C for 4 hrs, then placed in a muffle furnace and calcined at 650°C for 2 hrs and at 900°C for 2 hrs to obtain a finished catalyst. The composition of the catalyst is shown in Table 1.

100 mL of the catalyst was put into a reactor for performance evaluation under the conditions of an absolute pressure of 70 kPa, a liquid hourly space velocity of 1.0 h⁻¹, a temperature of 620°C, and a steam/ethylbenzene ratio of 0.8 (wt). The test results after the 100-hours reaction are listed in Table 1, and the test results after the 1000-hours reaction are listed in Table 2.

**Table 1: Catalyst compositions, properties and evaluation results of Examples and Comparative Examples**

| Catalyst No. | Catalyst Composition (parts) | | | | | | | Ratio "(%) | H₂-TPR main peak position(°C) | Ethylbenzene conversion rate *(%) | Styrene selectivity *(%) |
|---|---|---|---|---|---|---|---|---|---|---|---|
| | Fe₂O₃ | K₂O | CeO₂ | MoO₃ | CaO | Na₂O | TiO₂ | | | | |
| Ex. 1 | 71.5 | 11.2 | 10.5 | 2.8 | 3.2 | 0.8 | - | 78 | 576 | 76.1 | 96.5 |
| Ex. 2 | 62.8 | 13.6 | 11.6 | 4.8 | 5.4 | 1.8 | - | 80 | 582 | 76.7 | 95.9 |
| Ex. 3 | 69.4 | 10.8 | 13.8 | 3.6 | 1.5 | 0.9 | - | 76 | 575 | 75.8 | 96.2 |
| Ex. 4 | 83.1 | 6.2 | 8.3 | 1.2 | 0.4 | 0.8 | - | 72 | 571 | 75.4 | 96.8 |
| Ex. 5 | 80.3 | 7.4 | 8.1 | 0.6 | 2.6 | 1.0 | - | 74 | 573 | 75.6 | 96.7 |
| Ex. 6 | 70.9 | 11.14 | 6.9 | 3.5 | 6.9 | 0.6 | 0.06 | 77 | 575 | 75.9 | 96.6 |
| Ex. 7 | 70.4 | 10.6 | 11.2 | 3.0 | 3.2 | 1.6 | - | 75 | 574 | 75.7 | 96.5 |
| Ex. 8 | 72.6 | 8.6 | 11.9 | 2.1 | 3.3 | 1.5 | - | 74 | 572 | 75.5 | 96.6 |
| Ex. 9 | 72.5 | 11.4 | 10.6 | 2.1 | 2.9 | 0.5 | - | 79 | 579 | 76.3 | 96.4 |
| Ex. 10 | 70.2 | 10.6 | 12.0 | 2.6 | 3.5 | 1.1 | - | 77 | 576 | 76.0 | 96.3 |
| Ex. 11 | 74.3 | 9.5 | 11.2 | 1.8 | 2.7 | 0.5 | - | 75 | 574 | 75.8 | 96.5 |
| Ex. 12 | 71.5 | 11.2 | 10.5 | 2.8 | 3.2 | 0.8 | - | 74 | 573 | 75.7 | 96.4 |
| Ex. 13 | 71.5 | 11.2 | 10.5 | 2.8 | 3.2 | 0.8 | - | 67 | 570 | 75.0 | 96.7 |
| Ex. 14 | 71.5 | 11.2 | 10.5 | 2.8 | 3.2 | 0.8 | - | 70 | 571 | 75.2 | 96.2 |
| Ex. 15 | 71.5 | 11.2 | 10.5 | 2.8 | 3.2 | - | - | 77 | 575 | 75.6 | 95.5 |
| Comp. 1 | 71.5 | 11.2 | 10.5 | 2.8 | 4.0 | - | - | 16 | 545 | 72.7 | 96.4 |
| Comp. 2 | 61.0 | 11.2 | 21.0 | 2.8 | 3.2 | 0.8 | | 14 | 541 | 67.3 | 91.2 |
| Comp. 3 | 71.5 | 11.2 | 10.5 | 2.8 | 3.2 | 0.8 | | 18 | 546 | 72.8 | 95.5 |
| Comp. 4 | 71.5 | 11.2 | 10.5 | 2.8 | 3.2 | 0.8 | | 16 | 544 | 70.4 | 96.5 |
| Comp. 5*** | 68.5 | 11.2 | 10.5 | 2.8 | 3.2 | 0.8 | | 37 | 553 | 73.0 | 95.1 |

| | | | | | | | | | | | |
|---|---|---|---|---|---|---|---|---|---|---|---|
| Note: Ex.: Example; Comp.: Comparative Example *Ethylbenzene conversion rate and styrene selectivity for 100-hours reaction at steam/ethylbenzene ratio of 0.8; **Ratio: the ratio of the area of CeO₂ (100) exposed crystal face to the area of CeO₂ total exposed crystal face of the catalyst; ***Catalyst also contained 3.0 parts of Pr₂O₃. | | | | | | | | | | | |

**Table 2: Catalyst stability evaluation results of Examples and Comparative Examples**

| Catalyst No. | | 100-hours reaction | 1000-hours reaction |
|---|---|---|---|
| Ex. 1 | Ethylbenzene conversion rate (%) | 76.1 | 74.1 |
| | Styrene selectivity (%) | 96.5 | 96.6 |
| Ex. 7 | Ethylbenzene conversion rate (%) | 75.7 | 73.6 |
| | Styrene selectivity (%) | 96.5 | 96.7 |
| Ex. 12 | Ethylbenzene conversion rate (%) | 75.7 | 73.7 |
| | Styrene selectivity (%) | 96.4 | 96.5 |
| Ex. 14 | Ethylbenzene conversion rate (%) | 75.2 | 72.5 |
| | Styrene selectivity (%) | 96.2 | 96.4 |
| Comp. 1 | Ethylbenzene conversion rate (%) | 72.7 | 69.2 |
| | Styrene selectivity (%) | 96.4 | 96.5 |
| Comp. 4 | Ethylbenzene conversion rate (%) | 70.4 | 66.3 |
| | Styrene selectivity (%) | 96.5 | 96.5 |

100 mL of the catalyst was put into a reactor, and the performance evaluation was performed under the conditions of an absolute pressure of 70 kPa, a liquid hourly space velocity of 1.0 h⁻¹, a temperature of 620°C, and a steam/ethylbenzene ratio of 2 (wt). The test results after the 100-hours reaction are listed in Table 3.

**Table 3: Performance evaluation result of the catalyst of Example 1 under the condition of a steam/ethylbenzene ratio of 2 (wt)**

| | 100-hours reaction |
|---|---|
| Ethylbenzene conversion rate (%) | 85.2 |
| Styrene selectivity (%) | 97.6 |

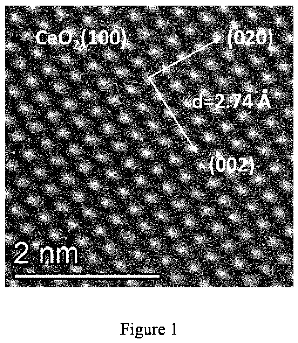

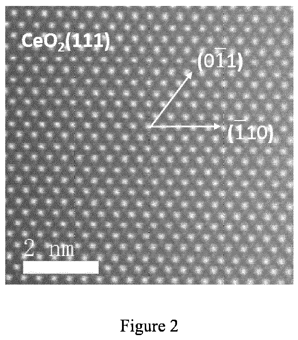

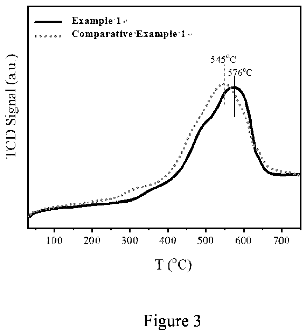

It can be seen from Figures 1, 2 and 3 that the main exposed crystal face of CeO₂ in the catalyst of Example 1 was the CeO₂ (100) crystal face. The main exposed crystal face of CeO₂ in the catalyst of Comparative Example 1 was the CeO₂ (111) crystal face, and the CeO₂ (111) crystal face comprised 52% of the total exposed crystal face of CeO₂. The H₂-TPR main reduction peak position of the catalyst of Example 1 was 576°C, which is significantly higher than the H₂-TPR main reduction peak position of the catalyst of Comparative Example 1, which was 545°C. Combining the results in Table 1 and Table 2, it can be seen that the catalysts of the present invention had high activity and good stability. After the dehydrogenation reaction had been run for a long time of 1000 hours, the ethylbenzene conversion rate did not significantly decrease.

The preferred embodiments of the present invention have been described in detail above, but the present invention is not limited thereto. Within the scope of the technical concept of the present invention, various simple modifications can be made to the technical solution of the present invention, including the combination of various technical features in any other suitable manner. These simple modifications and combinations should also be regarded as the disclosed content of the present invention and all belong to the protection scope of the present invention.

## Claims

1. A catalyst, **characterized in that** the composition of the catalyst is calculated as oxide, the catalyst contains Fe₂O₃, K₂O, CeO₂, MoO₃, and CaO; wherein the area of CeO₂ (100) exposed crystal face comprises 43% or higher, for instance 50% or higher, preferably 60% or higher, more preferably 70% or higher of the area of CeO₂ total exposed crystal face.

2. The catalyst according to any one of the preceding claims, **characterized in that** the H₂-TPR main reduction peak position of the catalyst is 570 - 620°C, such as 570°C, 580°C, 585°C, 590°C, 595°C, 600°C, 605°C, 610°C, 615°C, 620°C.

3. The catalyst according to any one of the preceding claims, **characterized in that**, the catalyst includes the following components in mass fraction based on the total mass of the catalyst:
(a) 60%-85% of Fe₂O₃;
(b) 6%-14% of K₂O;
(c) 6%-14% of CeO₂;
(d) 0.5%-5% of MoO₃;
(e) 0.3%-7% of CaO.

4. The catalyst according to claim 3, **characterized in that** the catalyst, based on the total mass of the catalyst, in mass fraction, contains 0.01%-2.0% Na₂O.

5. The catalyst according to claim 3, **characterized in that** the catalyst, based on the total mass of the catalyst, in mass fraction, contains 0.01%-2.0% Na₂O, the catalyst further contains 0.01%-2.0% other metal oxide(s), such as TiO₂.

6. The catalyst according to any one of the preceding claims, **characterized in that**, the catalyst is used in the ethylbenzene dehydrogenation, preferably the ethylbenzene dehydrogenation in a low steam/ethylbenzene ratio.

7. A method of preparing the catalyst according to any one of the preceding claims, **characterized in that** said method comprises the following steps:
mixing a Fe source, a Ce source, a Mo source, a Ca source, optionally a first K source, and optionally a pore-forming agent evenly, then adding an alkaline solution, letting stand for reaction, shaping, and calcining to produce the catalyst;
wherein, the K₂O in the catalyst derives from the first K source and/or the alkaline solution, for example, at least 50% of the K₂O in the catalyst derives from an alkaline solution containing K, and the remaining part derives from the first K source; or the K₂O in the catalyst can also all derive from an alkaline solution containing K instead of using the first K source.

8. The preparation method according to any one of the preceding claims, **characterized in that** the alkaline solution is an aqueous solution of potassium hydroxide and/or sodium hydroxide, preferably an aqueous solution of sodium hydroxide and potassium hydroxide, the mass ratio of sodium hydroxide as Na₂O to potassium hydroxide as K₂O is preferably 1:2-23; the OH group concentration of the alkaline solution is 1 mol/L - 8 mol/L;
and/or, the alkaline solution is an aqueous solution of sodium hydroxide and potassium hydroxide, the mass ratio of sodium hydroxide as Na₂O to potassium hydroxide as K₂O is preferably 1:2-23.

9. The preparation method according to any one of the preceding claims, **characterized in that**, at leaat a part of the K₂O in the catalyst derives from the alkaline solution; preferably, at least 50% of the K₂O in the catalyst derives from an alkaline solution containing K, and the remaining part derives from the first K source.

10. The preparation method according to any one of the preceding claims, **characterized in that** a raw material further contains a Ti source; the Ti source together with the Fe source, the Ce source, the Mo source, the Ca source, optionally the first K source, and optionally the pore-forming agent is mixed evenly and added; the Ti source is added in form of titanium salt or oxide; preferably, the titanium salt is any one or both of titanium tetrachloride or titanium tetrabromide.

11. The preparation method according to any one of the preceding claims, **characterized in that** the condition for letting stand for reaction comprises letting stand for reaction at 120-180°C for 12-48 hours.

12. The preparation method according to any one of the preceding claims, **characterized in that** the calcining temperature is 600-1000°C, the calcining time is 2-8 hours;
for example a two-step calcining is used, for example but not limited to calcining at 600-800°C for 2-4 hours, then calcining at 900-1000°C for 2-4 hours.

13. The preparation method according to any one of the preceding claims, **characterized in that** the calcining is carried out in a muffle furnace.

14. The preparation method according to any one of the preceding claims, **characterized in that** a shaped material is firstly dried and then calcined, the drying temperature is 50-200°C, the drying time is 1-24 hours.

15. The preparation method according to any one of the preceding claims, **characterized in that** the reaction is carried out under an increased pressure, for example 2-20 atm (gauge pressure).

16. The preparation method according to any one of the preceding claims, **characterized in that** the Ce source is added in form of cerium salt, for example the Ce source is cerium nitrate;
and/or, the Fe source is added in form of oxide Fe₂O₃, for example the Fe source is preferably selected from iron oxide red and/or iron oxide yellow, more preferably a composition of iron oxide red and iron oxide yellow, wherein the mass ratio of iron oxide red to iron oxide yellow as Fe₂O₃ is 1.0-3.5:1; and/or, the first K source is added in form of potassium salt, the first K source is added in form of potassium salt; the potassium salt is any one or more of potassium carbonate, potassium nitrate, and potassium bicarbonate;
and/or, the Mo source is added in form of molybdenum salt or oxide; the molybdenum salt is ammonium molybdate;
and/or, the calcium source is added in form of oxide or hydroxide;
optionally, the pore-forming agent is any one or more of activated carbon, graphite, sodium carboxymethylcellulose and polystyrene microsphere, and the pore-forming agent is added in an amount of 0-5% of the catalyst mass;
optionally, in the preparation method of the catalyst, the Fe source, the Ce source, the Mo source, the Ca source, the first K source, and the pore-forming agent are all added in form of solid phase powder.

17. Use of the catalyst according to any one of the preceding claims or the catalyst prepared with the preparation method according to any one of the preceding claims for producing styrene in the ethylbenzene dehydrogenation.

18. Use according to the preceding claim 17, **characterized in that** the catalyst is suitable for the ethylbenzene dehydrogenation in a low steam/ethylbenzene ratio; the low steam/ethylbenzene ratio is 1.3 or less, preferably 0.7-1.3.

19. Use according to the preceding claim 17, **characterized in that** the catalyst is suitable for the production of styrene with a steam/ethylbenzene ratio of 2.0 or less.

20. Use according to any one of the preceding claims, **characterized in that** said use comprises: a raw material gas containing ethylbenzene contacts with the catalyst in the presence of water vapor to perform a dehydrogenation reaction to produce a styrene-containing product;
water is pre-heated into water vapor before entering the reactor and thoroughly mixed with the raw material gas;
the temperature of the dehydrogenation reaction is 570-640°C;
the pressure of the dehydrogenation reaction is an absolute pressure of 20-100kPa;
the weight hourly space velocity of ethylbenzene is 0.2-2.0 h⁻¹.
